# EUROPEAN PATENT SPECIFICATION

(11) **EP 4 208 226 B1**
(45) Date of publication and mention of the grant of the patent: **18.02.2026**
(21) Application number: 21773002.7
(22) Date of filing: 31.08.2021
(51) Int. Cl.: A61M 11/02, A61M 15/00

(54) **DRUG DELIVERY DEVICES WITH PROPELLANT**
ARZNEIMITTELABGABEVORRICHTUNGEN MIT TREIBMITTEL
DISPOSITIFS D'ADMINISTRATION DE MÉDICAMENT COMPRENANT UN AGENT PROPULSEUR

(30) Priority: 01.09.2020 US 202063073000 P
(43) Date of publication of application: 12.07.2023
(60) Divisional application: 25209774.6
(73) Proprietor: Janssen Pharmaceutica NV, 2340 Beerse (BE)
(72) Inventor: RAMOS, David, Orange Park, Florida 32065 (US); KAPIL, Monica A., San Jose, California 95127 (US); LARSON, Chaley John, Horsham, Pennsylvania 19044 (US); SCHWARZ, Steven J., Spring House, Pennsylvania 19477 (US); HUBERT, Emma Louise, Milpitas, California 95035 (US); YAN, Hong, Milpitas, California 95035 (US)
(74) Representative: Carpmaels & Ransford LLP
(86) International application number: PCT/EP2021/074049
(87) International publication number: WO 2022/049083

(56) References cited:
- US-A1- 2002 092 523
- US-A1- 2010 258 118
- US-A1- 2011 126 830
- US-A1- 2020 179 378
- US-B1- 6 398 074

## Description

### FIELD

The present disclosure relates generally to drug delivery devices with propellant and drug products utilizing the same.

### BACKGROUND

There are many different ways in which a drug can be administered to a user. Depending on the drug, intranasal drug delivery can be one of the most effective ways to achieve desired clinical benefits in a timely manner and in a manner that is convenient and comfortable for a patient.

Intranasal drug administration is a non-invasive route for drug delivery. Since the nasal mucosa offers numerous benefits as a target tissue for drug delivery, a wide variety of drugs may be administered by intranasal systemic action. Moreover, intranasal drug delivery can avoid the risks and discomfort associated with other routes of drug delivery, such as intravenous drug delivery, and can allow for easy self-administration.

Generally, to maximize the efficacy of the drug through intranasal administration, the majority volume of the aerosolized dose of the drug needs to reach the correct region of the nasal cavity. As such, additional measures may need to be taken for effective intranasal drug delivery. For example, the user may need to have a clear nostril, tilt their head back at approximately 45°, close the opposite nostril, and then sniff gently while the dose of drug is administered. In order to coordinate these measures, and given that nasal administration is intimate, self-administration by the user may be desired. Further, due to the nasal cycle (alternating physiological partial congestion of the nasal turbinate to facilitate nasal function) or pathological congestion, one nostril is likely to provide a more effective drug delivery route than the other nostril at any given time. As such, it is desired that an equal dose of the drug be delivered to each nostril of the user to inhibit under-dosing of the drug.

Dual-dose intranasal drug delivery devices are available that are designed for self-administration of two distinct aerosolized sprays, one for each nostril, that together constitute one dose of drug. These devices require a series of operational steps that the user needs to properly carry out to effect optimal drug delivery through self-administration. One or more of the operational steps can require the user to have sufficient dexterity and/or strength to manually trigger delivery of the drug from the device by pushing on a trigger, a button, a plunger, or other mechanism. However, at least some users lack sufficient dexterity and/or strength to push on the trigger, the button, the plunger, or other mechanism of the drug, which may result in less drug than intended being delivered or no drug at all being delivered because drug delivery was not triggered with sufficient force.

Accordingly, there remains a need for improved nasal drug delivery devices.

US 6398074 B1 describes a reservoir for a fluid product containing a single dose, the reservoir comprising an air inlet and a product outlet, the air inlet comprising a product retention device to keep the product in the reservoir until dispensation of the product, and the product outlet being blocked, preferably in a sealed fashion, by a closing ball which is removed from its blocking position by the flow of air when the product is being dispensed.

US 2002/092523 A1 describes a medicament delivery device for administering a medicament to a user including a medicament reservoir and an entrance port and an exit port each disposed adjacent the reservoir. A gas chamber is disposed adjacent the entrance port.

US 2011/126830 A1 describes a delivery device for delivering a metered amount of substance, the delivery device comprising: a mouthpiece through which a user in use exhales; an outlet through which substance is in use delivered; a housing fluidly connected to the mouthpiece and the outlet, such, that exhalation by the user through the mouthpiece creates an air flow through the housing and from the outlet; and a substance-dispensing unit which is disposed within the housing and operative to dispense a metered amount of substance into an entraining air flow as created through the housing.

US 2020/179378 A1 describes pharmaceutical compositions, devices, their combinations, and their uses thereof for example in treating or preventing headaches.

US 2010/258118 A1 describes inhaler devices and bespoke pharmaceutical dry powder composition to be dispensed using such inhaler devices for pulmonary administration.

### SUMMARY

In general, drug delivery devices with propellant, drug products utilizing the same, and methods of using drug delivery devices with propellant are provided. The invention is defined by the appended claims.

In one aspect, a drug delivery device is provided that in one embodiment includes a tip configured to be positioned at a nose of a patient. The tip has an opening therein. The drug delivery device also includes a propellant chamber containing a propellant therein, a drug chamber containing a drug therein, a stopper positioned between the propellant chamber and the drug chamber, and an actuator configured to be actuated to cause the propellant to propel movement of the stopper in a direction toward the drug chamber and thereby propel the drug out of the opening.

The drug delivery device can have any number of variations. For example, the drug delivery device can also include a body that defines an internal cavity, and the internal cavity can define the propellant chamber and the drug chamber. For another example, the drug delivery device can also include a body that defines an internal cavity, the internal cavity can define the drug chamber and can have the stopper disposed therein, the drug delivery device can also include a container defining the propellant chamber, the drug delivery device can also include a tubular conduit extending between the container and the body, and the conduit can provide fluid communication between the internal cavity and the propellant chamber. For yet another example, the drug delivery device can also include a seal member that separates the tip from the drug chamber prior to the actuation of the actuator, and the propelling of the drug can be configured to break the seal member. For still another example, the tip, the propellant chamber, the drug chamber, and the stopper can be longitudinally aligned with one another. For yet another example, the propellant chamber and the drug chamber can not be longitudinally aligned with one another. For still another example, the propellant can include at least one of a gas and a material substance configured to produce energy that causes movement of the drug out of the opening.

For another example, the drug delivery device can also include a seal member that separates the propellant chamber into a first area, containing a first component of the propellant, and a second area, containing a second component of the propellant, prior to the actuation of the actuator such that the first and second components of the propellant are separated from one another prior to the actuation of the actuator. The actuation of the actuator can be configured to automatically break the seal member, and/or the first component can include vinegar and the second component can include sodium bicarbonate.

For still another example, the drug can be one of ketamine, esketamine, naloxone, and sumatriptan.

In another aspect, a drug product is provided that in one embodiment includes a drug product disposed in a drug delivery device. The drug delivery device includes a tip configured to be positioned at a nose of a patient. The tip has an opening therein. The drug delivery device also includes a propellant chamber containing a propellant therein, a drug chamber containing the drug product therein, a stopper positioned between the propellant chamber and the drug chamber, and an actuator configured to be actuated to cause the propellant to propel movement of the stopper in a direction toward the drug chamber and thereby propel the drug product out of the opening. The drug product is one of ketamine, esketamine, naloxone, and sumatriptan. The drug delivery device can have any number of variations.

**In** another aspect, a drug delivery method is provided that in one embodiment includes actuating an actuator of a drug delivery device and thereby cause a propellant in a propellant chamber to propel a stopper, which is positioned between the propellant chamber and a drug chamber of the drug delivery device, to move toward the drug chamber and thereby cause a drug contained in the drug chamber to spray out an opening of a tip of the drug delivery device that is positioned at a nose of a patient.

The drug delivery method can vary in any number of ways. For example, the actuation of the actuator can cause the propellant to pass through a tubular conduit that extends between a container containing the propellant chamber and an internal cavity defined by a body of the drug delivery device. For another example, the actuation of the actuator can cause the drug to break a seal member that separates the tip from the drug chamber prior to the actuation of the actuator. For still another example, the propellant can include at least one of a gas and a material substance configured to produce energy that causes movement of the drug out of the opening.

For yet another example, the actuation of the actuator can cause a chemical reaction between a first component of the propellant and a second component of the propellant. The first component can include vinegar, and the second component can include sodium bicarbonate.

For still another example, the drug can be one of ketamine, esketamine, naloxone, and sumatriptan.

In another embodiment, a drug delivery method includes coupling a drug holder to a tubular coupling mechanism of a drug delivery device. The drug holder contains a drug therein. The drug delivery method also includes, after coupling the drug holder to the tubular coupling mechanism, actuating an actuator of the drug delivery device and thereby cause a propellant in a propellant chamber to propel the drug out an opening of a tip of the drug delivery device that is positioned in a nose of a patient.

The drug delivery method can have any number of variations. For example, the propellant can be a gas. For another example, the drug holder can be a vial. For yet another example, the drug holder can be a blow-fill-seal capsule. For still another example, the drug delivery method can also include, after coupling the drug holder to the tubular coupling mechanism and before actuating the actuator, pumping a pumping mechanism of the drug delivery device and thereby pressurize a pumping chamber of the drug delivery device, and the actuation of the actuator can cause the propellant chamber to become in fluid communication with the pumping chamber. For another example, the actuation of the actuator can cause the propellant and the drug to swirl in the swirl chamber. For still another example, coupling the drug holder to the tubular coupling mechanism can cause the coupling member to break a seal member that provides a fluid tight seal of the drug holder. For another example, the drug can be one of ketamine, esketamine, naloxone, and sumatriptan.

In another aspect, a drug delivery system is provided that in one embodiment includes a drug holder containing a drug therein, and a drug delivery device. The drug delivery device includes a tip configured to be positioned in a nose of a patient. The tip has an opening therein. The drug delivery device also includes a tubular coupling mechanism configured to releasably couple to the drug holder containing the drug therein, a propellant chamber configured to contain a propellant therein, and an actuator configured to be actuated to cause the propellant to propel the drug out of the opening of the drug delivery device.

The drug delivery system can have any number of variations. For example, the propellant can be a gas. For another example the drug holder can be a vial. For yet another example, the drug holder can be a blow-fill-seal capsule. For still another example, the drug delivery device can also include a pumping chamber in fluid communication with the opening and with the coupling mechanism, the drug delivery device can also include a pumping mechanism configured to be pumped to pressurize the pumping chamber, and the actuation of the actuator can be configured to cause the propellant chamber to become in fluid communication with the pumping chamber. For another example, the drug delivery device can also include a swirl chamber in fluid communication with the opening and with the coupling mechanism, and the actuation of the actuator can be configured to cause the propellant chamber to become in fluid communication with the pumping chamber. For yet another example, the drug delivery device can also include a swirl chamber in fluid communication with the opening and with the coupling mechanism, and, with the drug holder coupled to the coupling mechanism, the actuation of the actuator can be configured to cause the propellant and the drug to swirl in the swirl chamber.

For another example, the drug holder can include a seal member that provides a fluid tight seal of the drug holder, and the seal member can be configured to be automatically broken by the coupling mechanism in response to the drug holder being coupled to the coupling mechanism. The coupling mechanism can include a pointed tip configured to break the seal member. The coupling mechanism can include a sharp edge configured to break the seal member.

For yet another example, the drug can be one of ketamine, esketamine, naloxone, and sumatriptan.

In another aspect, a drug product is provided that in one embodiment includes a drug product disposed in a drug delivery device. The drug delivery device includes a tip configured to be positioned in a nose of a patient. The tip has an opening therein. The drug delivery device also includes a tubular coupling mechanism configured to releasably couple to a drug holder containing the drug product therein, a propellant chamber configured to contain a propellant therein, and an actuator configured to be actuated to cause the propellant to propel the drug product out of the opening of the drug delivery device. The drug product is one of ketamine, esketamine, naloxone, and sumatriptan. The drug delivery device can have any number of variations.

### BRIEF DESCRIPTION OF DRAWINGS

The present invention is described by way of reference to the accompanying figures which are as follows:
FIG. 1 is a block diagram of one embodiment of a drug delivery device;
FIG. 2 is a perspective view of one embodiment of the drug delivery device of FIG. 1;
FIG. 3 is a perspective view of one embodiment of a clip configured to couple together two drug delivery devices;
FIG. 4 is a side cross-sectional view of another embodiment of the drug delivery device of FIG. 1;
FIG. 5 is a side view of yet another embodiment of the drug delivery device of FIG. 1;
FIG. 6 is a side view of still another embodiment of the drug delivery device of FIG. 1;
FIG. 7 is a side cross-sectional view of another embodiment of the drug delivery device of FIG. 1;
FIG. 8 is a side cross-sectional view of still another embodiment of the drug delivery device of FIG. 1;
FIG. 9 is a side view of one embodiment of a handle for the drug delivery device of FIG. 8;
FIG. 10 is a side view of another embodiment of a handle for the drug delivery device of FIG. 8;
FIG. 11 is a side cross-sectional view of another embodiment of the drug delivery device of FIG. 1;
FIG. 12 is a perspective view of still another embodiment of the drug delivery device of FIG. 1;
FIG. 13 is a perspective view of the drug delivery device of FIG. 12 with one embodiment of a drug holder coupled thereto and with one embodiment of a protective member or cap coupled thereto;
FIG. 14 is a perspective view of the drug delivery device and the drug holder of FIG. 13 with the protective member or cap removed from the drug delivery device;
FIG. 15 is a schematic view of flow in a swirl chamber of the drug delivery device of FIG. 14;
FIG. 16 is a side cross-sectional view of still another embodiment of the drug delivery device of FIG. 1;
FIG. 17 is a side cross-sectional view of a first actuation of the drug delivery device of FIG. 16;
FIG. 18 is a side cross-sectional view of a second actuation of the drug delivery device of FIG. 17;
FIG. 19 is a side cross-sectional view of yet another embodiment of the drug delivery device of FIG. 1;
FIG. 20 is a side cross-sectional view of a first actuation of the drug delivery device of FIG. 19;
FIG. 21 is a side cross-sectional view of a second actuation of the drug delivery device of FIG. 20;
FIG. 22 is a side cross-sectional view of a portion of the drug delivery devices of FIGS. 16 and 19;
FIG. 23 is a side cross-sectional view of a portion of the drug delivery devices of FIG. 22 during a first portion of a first stage of operation;
FIG. 24 is a side cross-sectional view of the portion of the drug delivery devices of FIG. 23 during a second portion of the first stage of operation;
FIG. 25 is a side cross-sectional view of the portion of the drug delivery devices of FIG. 24 during a third portion of the first stage of operation;
FIG. 26 is a side cross-sectional view of yet another embodiment of the drug delivery device of FIG. 1;
FIG. 27 is a side cross-sectional view of a first actuation of the drug delivery device of FIG. 26;
FIG. 28 is a side cross-sectional view of an intermediate configuration of the drug delivery device of FIG. 27;
FIG. 29 is a side cross-sectional view of a second actuation of the drug delivery device of FIG. 28; and
FIG. 30 is a side cross-sectional view of an end configuration of the drug delivery device of FIG. 29.

### DETAILED DESCRIPTION

Certain exemplary embodiments will now be described to provide an overall understanding of the principles of the structure, function, manufacture, and use of the devices, systems, and methods disclosed herein. One or more examples of these embodiments are illustrated in the accompanying drawings. A person skilled in the art will understand that the devices, systems, and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. A person skilled in the art will appreciate that a dimension may not be a precise value but nevertheless be considered to be at about that value due to any number of factors such as manufacturing tolerances and sensitivity of measurement equipment. Sizes and shapes of the systems and devices, and the components thereof, can depend at least on the size and shape of components with which the systems and devices will be used.

Various exemplary drug delivery devices with propellant, drug products utilizing the same, and methods of using drug delivery devices with propellant are provided. In general, a nasal drug delivery device is configured to expel a drug into a nose of a patient and to drive delivery of the drug using a propellant. In an exemplary embodiment, the propellant is non-mechanical and includes a gas, e.g., air, carbon dioxide, etc., and/or at least one material substance configured to produce energy that causes movement of the drug out of an opening of the drug delivery device. Using a propellant to propel the drug out of the drug delivery device may help ensure that the complete intended amount of the drug is delivered from the drug delivery device because the propellant can provide a predictable, sufficient amount of force to cause expulsion of the drug from the drug delivery device.

The drug to be delivered using a drug delivery device as described herein can be any of a variety of drugs. Examples of drugs that can be delivered using a drug delivery device as described herein include antibodies (such as monoclonal antibodies), hormones, antitoxins, substances for the control of pain, substances for the control of thrombosis, substances for the control of infection, peptides, proteins, human insulin or a human insulin analogue or derivative, polysaccharide, DNA, RNA, enzymes, oligonucleotides, antiallergics, antihistamines, anti-inflammatories, corticosteroids, disease modifying anti-rheumatic drugs, erythropoietin, and vaccines. Examples of drugs that can be delivered using a drug delivery device as described herein include ketamine (e.g., Ketalar^{®}), esketamine (e.g., Spravato^{®}, Ketanest^{®}, and Ketanest-S^{®}), naloxone (e.g., Narcan^{®}), and sumatriptan (e.g., Imitrex^{®}).

A drug delivery device configured to expel a drug into a nose of a patient and to drive delivery of the drug using a propellant can have a variety of configurations. In general, the drug delivery device is configured to deliver a drug to a patient, where the drug is provided in a defined dosage form within the drug delivery device.

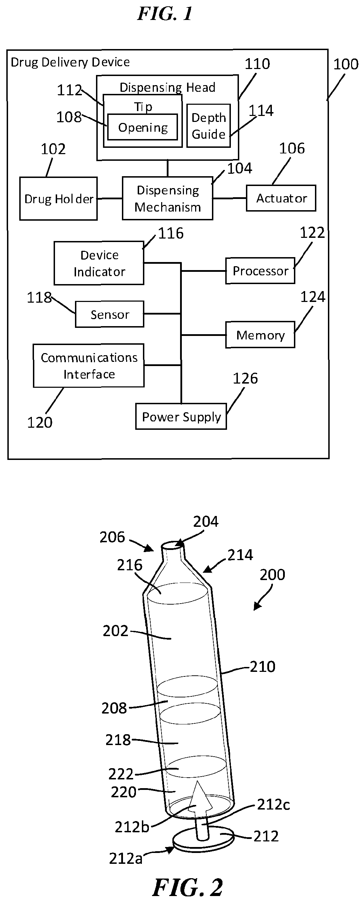

FIG. 1 illustrates one embodiment of a drug delivery device 100 configured to expel a drug into a nose of a patient and to drive delivery of the drug using a propellant. As will be appreciated by a person skilled in the art, the drug delivery device 100 can include different features in different embodiments depending upon various requirements, such as the type of drug, typical dosage(s) of the drug, safety requirements in various jurisdictions, whether the device is powered, etc.

The drug delivery device 100 includes a drug holder 102 configured to contain a drug therein for delivery from the device 100 to a patient. The drug holder 102 can have a variety of configurations, as discussed further below.

The drug delivery device 100 also includes a dispensing mechanism 104 that is operatively coupled to the drug holder 102 and configured to drive the drug out of device 100 from the drug holder 102. The dispensing mechanism 104 in this illustrated embodiment includes a propellant. The propellant can have a variety of configurations, as discussed further below. The dispensing mechanism 104 can include additional component(s). For example, the dispensing mechanism 104 can include a plunger configured to be driven by the propellant to push the drug out of the drug holder 102. For another example, the dispensing mechanism 104 can include a heating mechanism configured to heat the propellant to cause the propellant to drive the drug out of device 100 from the drug holder 102.

The drug delivery device 100 also includes an actuator 106 configured to be actuated by a user to cause the dispensing mechanism 104 to begin delivering a dose of the drug through an opening or nozzle 108 in the drug delivery device 100. In an exemplary embodiment, the drug delivery device 100 is configured to be self-administered such that the user who actuates the actuator 106 is the patient receiving the drug from the drug delivery device 100. The actuator 106 can have a variety of configurations, as discussed further below. For example, the actuator 106 can include a pressable button. For another example, the actuator 106 can include a movable switch. For yet another example, the actuator 106 can include a squeezable body of the drug holder 102.

The opening 108 through which the drug exits the drug delivery device 100 is formed in a dispensing head 110 of the drug delivery device 100, for example, in a tip 112 of the dispensing head 110. The tip 112 is configured to be inserted into a nostril of a patient. In an exemplary embodiment, the tip 112 is configured to be inserted into a first nostril of the patient during a first stage of operation of the drug delivery device 100 and into a second nostril of the patient during a second stage of operation of the drug delivery device 100. The first and second stages of operation involve two separate actuations of the actuator 106, a first actuation corresponding to a first dose of the drug being delivered and a second actuation corresponding to a second dose of the drug being delivered. The dispensing head 110 includes a depth guide 114 configured to contact skin of the patient, e.g., between the patient's first and second nostrils, such that a longitudinal axis of the dispensing head 110 is substantially aligned with a longitudinal axis of the nostril in which the tip 112 is inserted. A person skilled in the art will appreciate that the longitudinal axes may not be precisely aligned but nevertheless be considered to be substantially aligned due to any number of factors, such as manufacturing tolerances and sensitivity of measurement equipment.

In an exemplary embodiment, the dispensing head 110 has a tapered shape in which the dispensing head 110 has a smaller diameter at its distal end than at its proximal end where the opening 108 is located. The opening 108 having a relatively small diameter facilitates spray of the drug out of the opening 108, as will be appreciated by a person skilled in the art. A spray chamber through which the drug is configured to pass before exiting the opening 108 is located within a proximal portion of the tapered dispensing head 110, distal to the opening 108. When the drug passes through the spray chamber at speed, the spray chamber facilitates production of a fine mist that exits through the opening 108 with a consistent spray pattern.

In some embodiments, the dispensing head 110 can include two tips 112 each having an opening 108 therein such that the drug delivery device 100 is configured to simultaneously deliver doses of drug into two nostrils in response to a single actuation of the actuator 106.

The drug delivery device 100 also includes a device indicator 116 configured to present information to a user about a status of the drug delivery device 100 and/or the drug contained in the drug holder 102. The device indicator 116 can be a visual indicator, such as a display screen, one or more lights, one or more colored and/or numbered markings, etc. Alternatively or in addition, the device indicator 116 can be an audio indicator configured to provide sound.

The drug delivery device 100 also includes a sensor 118 configured to sense information relating to the drug delivery device 100 and/or the drug contained in the drug holder 102. Examples of information that the sensor 118 can sense include environmental conditions (e.g., temperature, humidity, geographic location, time, etc.). The drug delivery device 100 can also include a communications interface 120 configured to communicate externally data which has been gathered by the sensor 118 about the drug delivery device 100 and/or the drug contained in the drug holder 102, which may facilitate analysis regarding the patient's treatment, patient compliance, use of the drug delivery device 100, etc.

In embodiments in which the drug delivery device 100 includes one or more electrical components, e.g., the device indicator 116 (which in some embodiments can be powered while in other embodiments not be powered), the sensor 118, the communications interface 120, a processor 122, a memory 124, etc., the drug delivery device 100 includes a power supply 126 configured to deliver electrical power to the one or more electrical components of the drug delivery device 100. The power supply 126 can be a source of power which is integral to drug delivery device 100 and/or can be a mechanism configured to connect the drug delivery device 100 to an external source of power. The processor 122 is configured to receive gathered data from the sensor 118 and to cause the data to be stored in the memory 124, to be indicated on the device indicator 116, and/or and to be communicated externally via the communications interface 120. The memory 124 is configured to store instructions that are configured to be executed by the processor 122 for the processor 122 to process information regarding the various electrical components with which the processor 122 is in communication.

As mentioned above, the drug delivery device 100 can include different features in different embodiments depending upon various requirements. For example, the drug delivery device 100 can omit any one or more of the depth guide 114, the device indicator 116, the sensor 118, the communications interface 120, the processor 122, the memory 124, and the power supply 126.

FIG. 2 illustrates an exemplary embodiment of the drug delivery device 100 of FIG. 1. FIG. 2 illustrates a drug delivery device 200 configured to expel a drug 202 into a nose of a patient and to drive delivery of the drug 202 using a propellant. The drug delivery device 200 in this illustrated embodiment is in the form of a syringe. However, unlike traditional syringes, the syringe 200 of FIG. 2 lacks a plunger. Instead, the drug 202 is configured to be expelled through an opening 204 in a tip 206 of the drug delivery device 200 in response to movement of a stopper 208 caused by a chemical reaction of the propellant.

The stopper 208 separates the drug 202 and the propellant. The stopper 208 is thus a fluid-tight member. The stopper 208 can be made from one or more materials configured to prevent the drug 202 and the propellant from passing through the stopper 208 and from damaging the stopper 208 by causing the stopper 208 to degrade during storage of the drug delivery device 200. For example, the stopper 208 can be made from one or more polymers, as will be appreciated by a person skilled in the art. The stopper 208 has a size and shape configured to allow the stopper 208 to be sealably seated within a body 210 of the drug delivery device 200. The stopper 208 therefore has a size and shape complementary to a size and shape of an interior cavity of the body 210 in which the drug 202, the stopper 208, and the propellant are all located at least prior to actuation of an actuator 212 of the drug delivery device 200. **In** this illustrated embodiment, the interior cavity of the body 210 has a cylindrical shape, and the stopper 208 also has a cylindrical shape, however other shapes are possible. After actuation of the actuator 212, as discussed further below, at least some of the drug 202 has exited the body 210. The stopper 208 being sealably seated in the body 210 prevents the drug 202 and the propellant from passing through a gap of space between an exterior surface of the stopper and an internal surface of the body 210 that defines the interior cavity. The stopper 208 being sealably seated in the body 210 also prevents any of the propellant from exiting the drug delivery device 200 through the opening 204. **In** other words, the stopper 208 is configured to keep the propellant from passing proximally past the stopper 208.

The stopper 208 also has a size and shape configured to prevent the stopper 208 from moving proximally enough, e.g., during or after drug delivery, to exit the drug delivery device 100 through the opening 204. The stopper 208 has a larger diameter than the opening 204 such that the stopper 208 is too large to fit through the opening 204. Further, a dispensing head 214 of the drug delivery device 200 that includes the tip 206 and the opening 204 has a size and shape at least at its distal end that, in cooperation with the size and shape of the stopper 208, is configured to prevent the stopper 208 from entering the dispensing head 214. The stopper 208 is configured to move proximally toward the dispending head 214 during drug delivery. However, it is undesirable for the stopper 208 to exit the drug delivery device 200 during or after drug delivery so the propellant cannot exit the drug delivery device 200 through the opening 204 during or after drug delivery. The dispensing head 214 and the stopper 208 cooperating to prevent passage of the stopper 208 into the dispensing head 214 thus allows the propellant to remain in the drug delivery device during and after drug delivery.

The dispensing head 214 has a tapered shape in which the dispensing head 214 has a smaller diameter at its distal end than at its proximal end where the opening 204 is located. The opening 204 having a relatively small diameter facilitates spray of the drug 202 out of the opening 204, as will be appreciated by a person skilled in the art. The dispensing head 214 includes a spray chamber, as discussed above.

The drug delivery device 200 includes a first seal member 216 that separates an internal cavity defined by the dispensing head 214 and the internal cavity defined by the body 210. The first seal member 216 is configured to provide a fluid tight seal so as to prevent the drug 202 from passing from the internal cavity defined by the body 210 and into the dispensing head 214 prior to actuation of the actuator 212. The first seal member 216 thus prevents the drug 202 from prematurely exiting the drug delivery device 200 through the opening 204. The seal provided by the first seal member 216 is configured to be broken by the drug 202 in response to actuation of the actuator 212. More particularly, the first seal member 216 is configured to be broken in response to a force exerted on the drug 202, and thus on the first seal member 216 by the drug 202, by the chemical reaction of the propellant that urges the stopper 208 proximally, and thus urges the drug 202 located proximal to the stopper 208, proximally. The first seal member 216 can have a variety of configurations, as will be appreciated by a person skilled in the art, such as by being a polymer layer or a foil layer. The first seal member 216 can be protected from accidental puncturing, piercing, or other breakage before intended use with a removable protective member or cap, such as a tamper evident (TE) seal, etc., configured to be removably seated on the dispensing head 214. **In** some embodiments, the first seal member 216 can be omitted and instead a removable protective member or cap can be provided that is removed just prior to use of the drug delivery device 200.

The propellant in this illustrated embodiment includes a first component 218 and a second component 220 that are each disposed within the internal cavity defined by the body 210. The first component 218 and the drug 202 are separated from one another by the stopper 208. After actuation of the actuator 212, substantially all of the drug 202 has exited the drug delivery device 200 through the opening 204. A person skilled in the art will appreciate that all of the drug 202 may not have been expelled from the drug delivery device 200 but nevertheless be considered to have substantially all been expelled from the drug delivery device 200 due to any number of factors, such as sensitivity of measurement equipment and a negligible amount of the drug 202 sticking or otherwise remaining on an interior surface of the dispensing head 214.

The first and second components 218, 220 are separated from one another, prior to actuation of the actuator 212, with a second seal member 222 disposed within the internal cavity defined by the body 210. The second seal member 222 is configured to provide a fluid tight seal so as to prevent the first and second components 218, 220 from coming into contact with one another prior to actuation of the actuator 212. The second seal member 222 thus prevents the propellant from prematurely propelling the drug 202 to exit the drug delivery device 200 through the opening 204. The seal provided by the second seal member 222 is configured to be broken in response to actuation of the actuator 212, as discussed further below. The second seal member 222 can have a variety of configurations, as will be appreciated by a person skilled in the art, such as by being a polymer layer or a foil layer.

The first and second components 218, 220 are configured to cause a chemical reaction when the first and second components 218, 220 come into contact with one another. As mentioned above, the chemical reaction is configured to exert a force on the stopper 208 to urge the stopper 208 proximally, and thus exert a force on the drug 202 to urge the drug 202 proximally and out of the opening 204. The first and second components 218, 220 can be a variety of materials. The amounts of the first and second components 218, 220 vary based on the materials and the amount of the drug 202 to be delivered. For example, the first component 218 can be vinegar, and the second component 220 can be sodium bicarbonate (baking soda), or vice versa. Vinegar and baking soda coming into contact with one another will produce carbon dioxide and water. The carbon dioxide as a gas expands in the portion of the internal cavity of the body 210 in which the vinegar and sodium bicarbonate have come into contact with one another. The limited amount of space in this portion of the internal cavity of the body 210 will cause enough pressurization to cause an "explosion" that moves the stopper 208 proximally and thus the drug 202 proximally. For another example, the first component 218 can be Mentos candy, which includes among other elements gum arabic and gelatin, and the second component 220 can be Diet Coke soda, which among other elements includes carbon dioxide, potassium benzoate, and aspartame, or vice versa. Similar to that discussed above regarding vinegar and baking soda, the Mentos candy and Diet Coke soda coming into contact with one another will produce an "explosion" that moves the stopper 208 proximally and thus the drug 202 proximally.

The actuator 212 is configured to physically break the second seal member 222 to allow the first and second components 218, 220 to come into contact with one another. The actuator 212 includes a base 212a and a piercing tip 212b that extends proximally from the base 212a. The actuator 212 is configured to be pushed proximally to actuate the drug delivery 200. **In** particular, an external, distal surface of the base 212a is configured to be pushed proximally, e.g., by being pushed thereon by a finger of a user's hand or by being otherwise pushed, to actuate the drug delivery 200.

The piercing tip 212b is configured to puncture or pierce through the second seal member 222. The piercing tip 212b is a proximally tapering cone with a pointed proximal tip configured to puncture or pierce through the second seal member 222, but the piercing tip 212b can have other configurations. For example, the piercing tip 212b can have a sharp proximal edge, e.g., around a perimeter of the piercing tip's proximal end. For another example, the piercing tip 212b can include a plurality of proximally tapering cones each with a pointed proximal tip configured to puncture or pierce through the second seal member 222. For another example, the piercing tip 212b can include a disc having a plurality sharp blades extending proximally therefrom that are each configured to puncture or pierce through the second seal member 222.

The piercing tip 212b is configured to puncture or pierce through the second seal member 222 in response to proximal movement of the actuator 212, e.g., in response to the base 212a of the actuator 212 being pushed proximally. A sufficient distance exists between a distal surface of the second seal member 22 and the piercing tip 212b prior to actuation of the actuator 212 to help prevent the piercing tip 212b from prematurely puncturing or piercing through the second seal member 222. The force that needs to be provided to the base 212a is relatively small since only a relatively small force is needed to cause the piercing tip 212b to puncture or pierce through the second seal member 222.

The actuator 212 and the body 210 are releasably adhered to one another prior to actuation of the actuator 212 to prevent the actuator 212, and hence the piercing tip 212b thereof, from moving proximally before a desired time of drug delivery. Premature puncturing or piercing of the second seal member 22 can thus be prevented. The pushing force applied to the base 212a is enough to detach the actuator 212 from the body 210. The actuator 212 and the base 212a can be releasably adhered to one another in any of a variety of ways, such as by using a light adhesive, one of the actuator 212 and the body 210 including a protrusion releasably seated in a depression formed in the other of the actuator 212 and the body 210, one of the actuator 212 and the body 210 including a clip that clips onto a protrusion extending from the other of the actuator 212 and the body 210, etc. **In** this illustrated embodiment, a distal surface of the piercing tip 212b is releasably attached to an interior distal surface of the body 210 prior to actuation of the actuator 212.

The body's distal end has a hole therein through which an elongate connector portion 212c of the actuator 212 extends such that the base 212a of the actuator 212 is located outside of the body 210 and the piercing tip 212b is located inside the body 210, e.g., in the internal cavity of the body 210. The elongate connector portion 212c slides in the hole during actuation, e.g., during pushing of the base 212a. The base 212a remains located outside of the body 210 before, during, and after drug delivery. The piercing tip 212b remains inside the body 210 before, during, and after drug delivery.

A fluid tight seal is provided between the elongate connector portion 212c and the body 210 to prevent the second component 220 from exiting the drug delivery device 200 distally before drug delivery and to prevent the first and second components 218, 220 from exiting the drug delivery device 200 distally during movement of the actuator 212 or after the second seal member 222 has been broken. The elongate connector portion 212c has a size and shape configured to allow the elongate connector portion 212c to be sealably and slidably seated within the hole in the body 210. **In** this illustrated embodiment, the hole has a circular shape, and the elongate connector portion 212c has a cylindrical shape, however other shapes are possible. A fourth seal member can be provided at the hole, e.g., an o-ring, etc., to help provide the seal at the hole in the body 210.

The actuator 212 can be made from one or more materials. At least the piercing tip 212b and the elongate connector portion 212c of the actuator 212 can be made from one or more materials configured to prevent the second component 220, prior to contact with the first component 218, and the first and second components 218, 220 in contact with one another, during and after drug delivery, from passing through either the piercing tip 212b or the elongate connector portion 212c and from damaging the piercing tip 212b or the elongate connector portion 212c by causing either the piercing tip 212b or the elongate connector portion 212c to degrade during storage of the drug delivery device 200. For example, at least the piercing tip 212b and the elongate connector portion 212c can be made from one or more polymers, as will be appreciated by a person skilled in the art.

Pressure applied to the actuator 212 in a proximal direction to trigger drug delivery, e.g., by a user pushing on the base 212a, will allow the chemical reaction of the first and second components 218, 220 to occur and cause proximal movement of the stopper 208 instead of distal movement of the actuator 212. The chemical reaction of the first and second components 218, 220 happens quickly enough when the first and second components 218, 220 come into contact with one another that the user will still be applying pressure to the actuator 212 when the chemical reaction occurs and causes pressurization in the internal cavity of the body 210. By the time the user releases the actuator 212, unless user error occurs by the user applying insufficient pressure to the actuator 212 to begin drug delivery, the chemical reaction will have already begun and can continue because the first and second components 218, 220 have come into contact with one another.

Before drug delivery, e.g., before the actuator 212 is pushed, the stopper 208 defines a proximal stop for the propellant, and the body 210 defines a distal stop for the propellant. **In** other words, the stopper 208 and the body 210 are configured to cooperate to keep the propellant contained within the drug delivery device 200 before drug delivery. After drug delivery, the stopper 208 defines the proximal stop for the propellant, and the actuator 212 defines the distal stop for the propellant. **In** other words, the stopper 208 and the actuator 212 are configured to cooperate to keep the propellant contained within the drug delivery device 200 after drug delivery. The drug delivery device 200 is therefore configured to keep the propellant safely contained therein before and after use of the drug delivery device 200. The body 210 and the base 212a of the actuator 212 can be configured to adhere to one another, e.g., a proximal surface of the base 212a adhered to an exterior distal surface of the body 210, after actuation of the actuator 212 to further help prevent the actuator 212 from moving distally in response to the chemical reaction of the first and second components 218, 220. The body 210 and the base 212a can be configured to adhere to one another in any of a variety of ways, such as by one of the body 210 and the base 212a including a protrusion configured to seat in a depression formed in the other of the body 210 and the base 212a, one of the body 210 and the base 212a including a clip that clips onto a protrusion extending from the other of the body 210 and the base 212a, etc.

In the illustrated embodiment of FIG. 2, the drug delivery device 200 is configured to deliver the drug 202 into only one nostril of a patient, e.g., with the tip 206 positioned in the one nostril. Two of the drug delivery devices 200 may therefore need to be used to deliver the drug 202 into each of the patient's nostrils, depending on one or more factors such as the amount of drug 202 in the drug delivery device 200, the amount of the drug 202 that the patient has been prescribed per dosing session, etc. In another embodiment, the drug delivery device 200 of FIG. 2 is configured to simultaneously deliver the drug 202 into both nostrils of a patient. For example, the drug delivery device 200 can include a second tip with a second hole through which the drug 202 is configured to exit the drug delivery device 200. The drug delivery device 200 can be actuated, e.g., by pushing the actuator 212, to cause the drug 202 to be propelled out of each of the two tips simultaneously. In some embodiments, the one tapered dispensing head 214 can include both of the tips. In other embodiments, the drug delivery device 200 can include two tapered dispensing heads with each of the dispensing heads including one of the two tips.

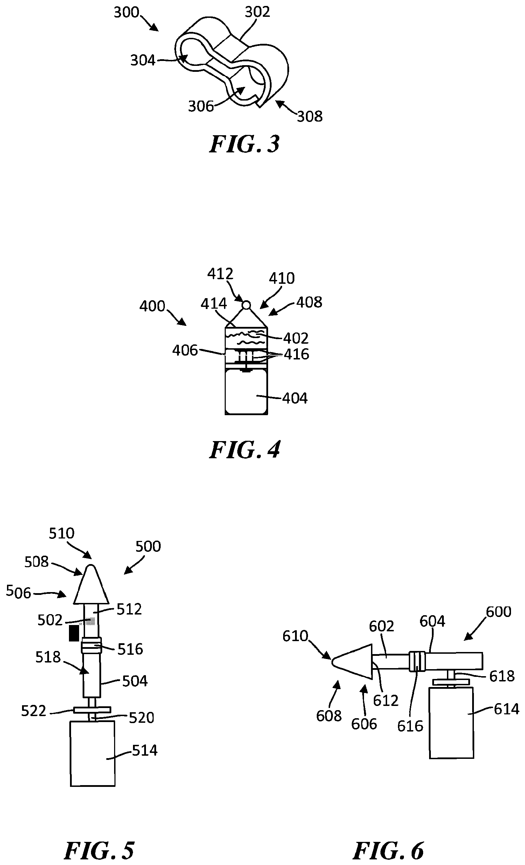

For another example of the drug delivery device 200 of FIG. 2 being configured to simultaneously deliver the drug 202 into both nostrils of a patient, two of the drug delivery devices 200 can be clipped together. FIG. 3 illustrates one embodiment of a clip 300 configured to clip two of the drug delivery devices 200 together. The clip 300 includes a body 302 that defines two cylindrical passages 304, 306. Each of the passages 304, 306 is configured to securely seat one of the drug delivery devices 200 therein. Each of the passages 304, 306 has a circumference just slightly larger than an outer circumference of the body 210 of the drug delivery device 200 to allow an interior surface of the body 302 that defines the passages 304, 306 to securely grip the drug delivery device 200, e.g., the body 210 thereof. Alternatively, each of the passages 304, 306 can have a circumference that is less than or equal to the outer circumference of the body 210 of the drug delivery device 200, and at least a portion of the interior surface of the body 302 that defines the passages 304, 306 can be formed of or have thereon a resilient material configured to securely grip the drug delivery device 200, e.g., the body 210 thereof. The interior surface of the body 302 that defines the passages 304, 306 can include a gripping feature configured to facilitate secure seating of the drug delivery device 200 therein. The gripping feature can have any of a variety of configurations, such as a textured surface, a rubber lining on the interior surface of the body 302, etc. The clip 300 in this illustrated embodiment includes a release mechanism 308 configured to facilitate coupling of the drug delivery devices 200 to the clip 300 and decoupling of the drug delivery devices 200 from the clip 300. Decoupling the drug delivery devices 200 from the clip 300 allows the clip 300 to be reused with other drug delivery devices same or different from the drug delivery devices 200. The release mechanism 308 is configured to move between a closed state, in which the clip's body 302 defines an enclosed perimeter, and an open state, in which the clip's body 302 has a broken or discontinuous perimeter. The release mechanism 308 is shown in the closed state in FIG. 3. The release mechanism 308 can have a variety of configurations. For example, as shown in this illustrated embodiment, the release mechanism 308 can include a hole formed through one end of the body 302 and a protrusion extending from the other end of the body 302 that is configured to releasably seat in the hole. For another example, the release mechanism 308 can include Velcro^{®} on each end of the body 302. For yet another example, the release mechanism 308 can include a magnet on each end of the body 302. For still another example, the release mechanism 308 can include a snap connection.

Referring again to FIG. 2, the actuator 212 in this illustrated embodiment is configured to move in response to a physical action taken by a user that results in the actuator 212 moving proximally relative to the body 210. In other embodiments, the actuator 212 can be configured to move in response to a non-physical action taken by a user that results in the actuator 212 moving proximally relative to the body 210. For example, the non-physical action can include a voice command. A user of such a voice-activated drug delivery device 200 may therefore position the tip 206 in a nostril (or two tips in two nostrils in some embodiments) and simply speak or otherwise make a predetermined voice command sound to begin drug delivery. Such a drug delivery device 200 may be easier for at least some users to hold and/or actuate since a finger need not be used to manually push the actuator 212.

In embodiments in which the actuator 212 is configured to move in response to a voice command, the actuator 212 can be operatively coupled to a processor of the drug delivery device 200. The processor can be operatively coupled to a sensor of the drug delivery device 200 that is configured to gather sound. In response to the sensor gathering sound that matches a predetermined sound or a predetermined pattern of sound, as determined by the processor, the processor can be configured to electrically drive the actuator 212 to move proximally and thereby begin drug delivery. The drug delivery device 200 can include a biasing member, such as a coil spring, a volute spring, etc., that biases the actuator 212 in a proximal direction such that the actuator 212 is configured to move proximally in response to force provided by the biasing member. The predetermined sound or the predetermined pattern of sound can be stored in a memory of the drug delivery device 200. The actuator 212 can be electrically driven in any of a variety of ways. For example, the drug delivery device 200 can include a locking mechanism configured to move between a locked state, in which the actuator 212 is held in an initial position, e.g., as shown in FIG. 2, and an unlocked state, in which the actuator 212 is free to move proximally relative to the body 210. The locking mechanism can have any of a variety of configurations. For example, the locking mechanism can include mechanical member configured to mechanically hold the biasing member with the actuator 212 in the locked state and configured to be moved by the processor in response to a verified voice command, e.g., by closing a switch in a circuit that causes the mechanical member to release its hold on the biasing member. For another example, the locking mechanism can include a first magnet coupled to the actuator 212 and a second magnet coupled to the body 210. The processor can be configured to cause the actuator 212 to rotate in response to a verified voice command, e.g., by closing a switch in a circuit that causes the actuator 212 to rotate, and thereby causing the first and second magnets to move out of magnetic range such that the actuator 212 is free to move proximally relative to the body 210 under the force of the biasing member.

Other embodiments of drug delivery devices described herein can similarly be configured to be voice activated.

**In** the illustrated embodiment of FIG. 2, the body 210 is rigid so as to not be able to be squeezed. The body 210 being unable to be squeezed may help prevent the stopper 208 from moving proximally prematurely before intended drug delivery in response to the body 210 being deformed by squeezing and/or may help prevent the first seal member 216 and/or the second seal member 222 from breaking prematurely before intended drug delivery in response to the body 210 being deformed by squeezing. **In** other embodiments, the body 210 is flexible so as to be able to be squeezed and deformed at least in an area of the body 210 adjacent to the second seal member 222. In such embodiments, the body 210 is configured to be squeezed in at least the area of the body 210 adjacent to the second seal member 222 to cause the second seal member 222 to break in response to the squeezing, thereby allowing the first and second components 218, 220 to cause a chemical reaction similar to that discussed above regarding the actuator 212 breaking the second seal member 222. The body 210 of the drug delivery device 200 can thus serve as the actuator, and the actuator 212 illustrated in FIG. 2 can be omitted.

In the illustrated embodiment of FIG. 2, the drug 202 and the first and second components 218, 220 of the propellant are separated from one another before actuation of the actuator 212 to begin drug delivery. In other embodiments, the drug 202 and the first component 218 are separated from the second component 220 before actuation of the actuator 212 to begin drug delivery but are not separated from each other before actuation of the actuator 212 to begin drug delivery. In such embodiments, the drug 202 and the first component 218 are contained in the same portion of the body 210 before actuation of the actuator 212 to begin drug delivery, and the stopper 208 is located above this portion of the body 210 that contains the drug and the first component 218 therein. In embodiments in which the body 210 is configured to be squeezed to break the seal member 222 separating the first and second components 218, 220, (or before the body 210 is squeezed, the drug 202 and the first component 218 are contained in the same portion of the body 210 before the body 210 is squeezed.

FIG. 4 illustrates another exemplary embodiment of the drug delivery device 100 of FIG. 1. FIG. 4 illustrates a drug delivery device 400 configured to expel a drug 402 into a nose of a patient and to drive delivery of the drug 402 using a propellant 404. The drug delivery device 400 of FIG. 4 is generally configured and used similar to the drug delivery device 200 of FIG. 2, e.g., includes a body 406, a dispensing head 408 including a tip 410 with an opening 412 therein, and a seal member 414 that separates an internal cavity defined by the dispensing head 408 and an internal cavity defined by the body 406. However, unlike the drug delivery device 200 of FIG. 2, the drug delivery device 400 of FIG. 4 includes first and second components of the propellant 404 that are not separated from one another before actuation of the drug delivery device's actuator to begin drug delivery. Additionally, the drug delivery device 400 of FIG. 4 includes a plunger 416 that separates the drug 402 and the propellant 404. The plunger 416 is configured to push the drug 402 proximally to be expelled out of the drug delivery device 400 through the opening 412. The actuator of the drug delivery device 400 includes the body 406 of the drug delivery device 400 that is configured to be squeezed at an area of the body 406 adjacent to the portion of the body's internal cavity that contains the propellant 404 therein. The squeezing of the body 406 is configured to cause the first and second components to mix and be agitated to trigger a chemical reaction of the first and second components of the propellant 404.

In the illustrated embodiment of FIG. 4, the drug delivery device 400 is configured to deliver the drug 402 into only one nostril of a patient, e.g., with the tip 410 positioned in the one nostril. In another embodiment, the drug delivery device 400 of FIG. 4 is configured to simultaneously deliver the drug 402 into both nostrils of a patient, similar to that discussed above regarding the drug delivery device 200 of FIG. 2.

FIG. 5 illustrates another exemplary embodiment of the drug delivery device 100 of FIG. 1. FIG. 5 illustrates a drug delivery device 500 configured to expel a drug 502 into a nose of a patient and to drive delivery of the drug 502 using a propellant. The drug delivery device 500 of FIG. 5 is generally configured and used similar to the drug delivery device 400 of FIG. 4, e.g., includes a body 504, a dispensing head 506 including a tip 508 with an opening 510 therein, and a seal member 512 that separates an internal cavity defined by the dispensing head 504 and an internal cavity defined by the body 502. However, unlike the drug delivery device 400 of FIG. 4, the propellant of the drug delivery device 500 of FIG. 5 is disposed in a container 514 located outside of the body 502 of the drug delivery device 500, and the drug delivery device 500 includes a stopper 516 configured to be driven proximally by the propellant similar to the stopper 208 of FIG. 2. A tubular conduit 520 extends proximally from the container 514 and is in fluid communication with the propellant in the container 514 and with a distal portion 518 of an internal cavity of the body 502 that is distal to the stopper 516. Propellant is configured to exit the container 514 and enter the distal portion 518 of the body's internal cavity through the conduit 520 and thereby drive the stopper 516 proximally to propel the drug 502 out of the opening 510. Moving the container 514 proximally relative to the 504 is configured to actuate the drug delivery device 500. A valve is located at a proximal end of the container 514. A user can put their pointing and middle fingers on a flange 522 at a distal end of the body 504 and can put a thumb on an external distal surface of the container 514. Pushing the thumb proximally toward the flange 522 can cause the container 514 to move and the propellant therein to be released from the container 514 in a proximal direction through the valve. The drug delivery device 500 can include a spacer located between the container 514 and the flange 522, e.g., at the conduit 520, that is configured to prevent premature actuation by locking the container 514 and the flange 522 in position relative to one another. The spacer can be removed by the user prior to desired actuation of the drug delivery device 500.

**In** some embodiments, the amount of the drug 502 and the amount of the propellant are such that the drug delivery device 500 is limited to a single drug delivery. **In** other embodiments, the amount of the drug 502 and the amount of the propellant are such that the drug delivery device 500 is configured to provide a plurality of drug deliveries. **In** such embodiments, the actuation of the drug delivery device 500 is configured to cause a predetermined amount of the propellant through the conduit 518 that is less than a total amount of propellant than at least initially contained in the container 514. The predetermined amount of the propellant causes delivery of a partial amount of the drug 502 in the drug delivery device 500. The last actuation of the drug delivery device 500 can result in substantially no drug 502 left in the drug delivery device 500.

In the illustrated embodiment of FIG. 5, the drug delivery device 500 is configured to deliver the drug 502 into only one nostril of a patient, e.g., with the tip 508 positioned in the one nostril. In another embodiment, the drug delivery device 500 of FIG. 5 is configured to simultaneously deliver the drug 502 into both nostrils of a patient, similar to that discussed above regarding the drug delivery device 200 of FIG. 2.

FIG. 6 illustrates another exemplary embodiment of the drug delivery device 100 of FIG. 1. FIG. 6 illustrates a drug delivery device 600 configured to expel a drug 602 into a nose of a patient and to drive delivery of the drug 602 using a propellant. The drug delivery device 600 of FIG. 6 is generally configured and used similar to the drug delivery device 500 of FIG. 5, e.g., includes a body 604, a dispensing head 606 including a tip 608 with an opening 610 therein, a seal member 612 that separates an internal cavity defined by the dispensing head 604 and an internal cavity defined by the body 602, a container 614 located outside of the body 604 and containing the propellant therein, a stopper 616, and a tubular conduit 618 extending between the container 614 and the body 604. However, unlike the drug delivery device 500 of FIG. 5, the container 614 and the conduit 618 are not linearly aligned with the body 604 of the drug delivery device 600 and thus are also not linearly aligned with the drug 602 contained in the body 604 and with the dispensing head 606 and the stopper 616. In the illustrated embodiment of FIG. 5, the container 514 and the conduit 518 are linearly aligned with the body 504 of the drug delivery device 500 and thus are also linearly aligned with the drug 502 contained in the body 504 and with the dispensing head 506 and the stopper 516. For different users, the position of the container 514, 614 relative to the body 504, 604 may make the drug delivery device 500, 600 easier for the user to hold and actuate.

In the illustrated embodiment of FIG. 6, the drug delivery device 600 is configured to deliver the drug 602 into only one nostril of a patient, e.g., with the tip 608 positioned in the one nostril. In another embodiment, the drug delivery device 600 of FIG. 6 is configured to simultaneously deliver the drug 602 into both nostrils of a patient, similar to that discussed above regarding the drug delivery device 200 of FIG. 2.

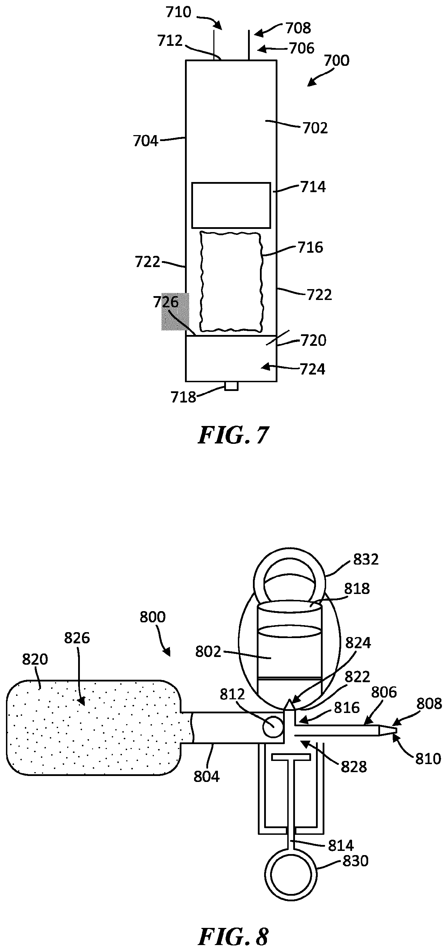

FIG. 7 illustrates another exemplary embodiment of the drug delivery device 100 of FIG. 1. FIG. 7 illustrates a drug delivery device 700 configured to expel a drug 702 into a nose of a patient and to drive delivery of the drug 702 using a propellant. The drug delivery device 700 of FIG. 7 is generally configured and used similar to the drug delivery device 200 of FIG. 2, e.g., includes a body 704, a dispensing head 706 including a tip 708 with an opening 710 therein, and a seal member 712 that separates an internal cavity defined by the dispensing head 706 and an internal cavity defined by the body 704. However, unlike the drug delivery device 200 of FIG. 2, the drug delivery device 700 of FIG. 7 includes a plunger 714 configured to push the drug 702 proximally to be expelled out of the drug delivery device 700 through the opening 710, and the propellant is contained within a bladder 716 disposed in the body 704. The propellant in this illustrated embodiment includes a gas configured to expand when heated. The bladder 716 is a flexible member configured to expand. The expansion of the bladder 716 is configured to press against the plunger 714 in a proximal direction and thereby cause the drug 702 to be delivered from the drug delivery device 700. The bladder 716 and the plunger 714 are separate elements in this illustrated embodiment, but in other embodiments the bladder 716 and the plunger 714 can be integrated together as a single element, e.g., with the plunger 714 attached to a proximal end of the expandable bladder 716. In other embodiments, the plunger 714 can be omitted, and the bladder 716 can be configured to be used as the actuator of the drug delivery device 700.

The actuator of the drug delivery device 700 includes a switch 718 of the drug delivery device 700 that is configured to be actuated, e.g., pressed or otherwise moved. The actuation of the switch 718 is configured to cause a processor 720 of the drug delivery device 700 to activate, e.g., turn on, a heating element 722 of the drug delivery device 700 that is operatively coupled to the bladder 716 and thus to the propellant contained in the bladder 716. Heat provided by the activated heating element 722 is configured to cause the expansion of the propellant and thus the expansion of the bladder 716 and the delivery of the drug 702 from the drug delivery device 700. The heating element 722 in this illustrated embodiment includes a heat coil coiled around the body 704 in a sidewall of the body 704 adjacent to the bladder 716 disposed in the body 704. However, the heating element 722 can have other configurations and can be otherwise coupled to the drug delivery device 700. For example, the heating element 722 can be in a sidewall of the body 704 adjacent to the bladder 716 disposed in the body 704 but have a form other than a heat coil, e.g., a heat cable, a positive temperature coefficient (PTC) heater, a resistive element configured to warm as current passes therethrough, etc. For another example, the bladder 716 can be formed of a material, such as a polymer, configured to heat up in response to a current provided by the heating element 722. For yet another example, the bladder 716 can be formed of a piezoelectric material configured to heat up in response to a vibration provided by the heating element 722.

The bladder 716 can be made from one or more materials configured to prevent the propellant from passing therethrough, that do not adversely affect the propellant (e.g., by causing the propellant to degrade during storage of the drug delivery device 700), and that are not adversely affected by being heated by the heating element 722. For example, the bladder 716 can be made from one or more polymers, as will be appreciated by a person skilled in the art.

The processor 720 is configured to deactivate, e.g., turn off, the heating element 722 to prevent any number of adverse consequences, such as the drug delivery device 700 becoming too hot to hold or to be properly disposed of after use, the heating element 722 becoming overheated, etc. The processor 720 can be configured to deactivate the heating element 722 in a variety of ways. For example, the processor 720 can be configured to deactivate the heating element 722 after a predetermined amount of time has passed since activation of the heating element 722. The amount of time that has passed since activation of the heating element 722 can be sensed by a sensor, e.g., a counter, a timer, etc., of the drug delivery device 700 that is in operative communication with the processor 720. The predetermined amount of time can be stored in a memory of the drug delivery device 700 that is in operative communication with the processor 720. The predetermined amount of time can correspond to a known amount of time that it takes for the propellant to become heated enough to expand and thereby cause drug delivery.

For another example, the processor 720 can be configured to deactivate the heating element 722 in response to drug delivery having been completed. The processor 720 can be configured to determine that drug delivery has been completed in a variety of ways, such as by analyzing data gathered by a sensor of the drug delivery device 700 that is in operative communication with the processor 720. For example, the sensor can include a Hall effect sensor configured to sense displacement of the plunger 714. A Hall effect sensor provides a reliable measurement of the displacement since a Hall effect sensor is not affected by the presence of dust particles, or other physical objects, which can obscure a line of sight of other sensors and thus affect the measurements. The displacement of the plunger 714 can be measured instead or additionally using, e.g., a motion sensor and/or a pressure sensor. For another example, the sensor can include a volumetric flow meter, e.g., a piston meter, an oval gear meter, etc., which enables the processor 720 to calculate a total volume of the drug 702 administered and can therefore be used to confirm delivery of the drug 702 from the drug delivery device 700. The volumetric flow meter can be positioned at or near the opening 710 of the drug delivery device 700.

For yet another example, the processor 720 can be configured to deactivate the heating element 722 in response to the first of a predetermined amount of time having passed since activation of the heating element 722 and a determination that drug delivery has been completed.

For still another example, a temperature sensor in communication with the processor 720 can be configured to sense temperature and to provide sensed temperature data to the processor 720. The processor 720 can be configured to deactivate the heating element 722 in response to the sensed temperature being above a predetermined threshold temperature. The temperature sensor can be configured to provide a plurality of sensed temperatures to the processor 720 while the heating element 722 is activated, such as a sensed temperature every one second, every two seconds, every five seconds, every ten seconds, etc.

The processor 720 in this illustrated embodiment is contained within an electronics compartment 724 in the body 704 of the drug delivery device 700. Other electronic components of the drug delivery device 700 can also be located in the electronics compartment 724. The electronics compartment 724 provides a fluid tight area within the drug delivery device 700 for electronics to help prevent any of the electronics therein from being damaged by fluid. A proximal surface 726 that defines a proximal side of the electronics compartment 724 defines a distal stop surface for the bladder 716. The distal stop surface helps control expansion of the bladder 716 in a proximal direction toward the plunger 714.

The processor 720 is on-board the drug delivery device 700 in this illustrated embodiment, but in other embodiments the processor 720 can be off-board from the drug delivery device 700 and be configured to electronically communicate with the heating element 722 wirelessly or via wired connection. The processor 720 being off-board from the drug delivery device 700 may help reduce cost of the drug delivery device 700 and/or may facilitate more environmentally friendly disposal of the drug delivery device 700.

**In** the illustrated embodiment of FIG. 7, the drug delivery device 700 is configured to deliver the drug 702 into only one nostril of a patient, e.g., with the tip 708 positioned in the one nostril. In another embodiment, the drug delivery device 700 of FIG. 7 is configured to simultaneously deliver the drug 702 into both nostrils of a patient, similar to that discussed above regarding the drug delivery device 200 of FIG. 2. In yet another embodiment, the switch 718 is configured to be actuated twice, with a first actuation of the switch 718 corresponding to a first stage of operation of the drug delivery device 700 with drug delivery into one nostril of a patient and a second, subsequent actuation of the switch 718 corresponding to a second stage of operation of the drug delivery device 700 with drug delivery into the other nostril of the patient. In such an embodiment, the heating element 722 is configured to provide heat for a first predetermined amount of time, as controlled by the processor 720, to cause the first stage of operation, then to be deactivated by the processor 720, and then to be reactivated by the processor 720 in response to the second actuation of the switch 718 after the drug delivery device 700 has been moved so the tip 708 is positioned in the patient's other nostril. In the first stage of operation, the bladder 716 expands to push the plunger 714 a first distance proximally. In the second stage of operation, the bladder 716 further expands to push the plunger 714 a second distance proximally.

FIG. 8 illustrates another exemplary embodiment of the drug delivery device 100 of FIG. 1. FIG. 8 illustrates a drug delivery device 800 configured to expel a drug 802 into a nose of a patient and to drive delivery of the drug 802 using a propellant 820. The drug delivery device 800 of FIG. 8 is generally configured and used similar to the drug delivery device 200 of FIG. 2, e.g., includes a body 804, a dispensing head 806 including a tip 808 with an opening 810 therein, and an actuator 812. However, unlike the drug delivery device 200 of FIG. 2, the drug delivery device 800 of FIG. 8 includes a pumping mechanism 814 configured to facilitate propellant-driven delivery of the drug 802 from the drug delivery device 800, as discussed further below.

Also, the drug delivery device 800 of FIG. 8 includes a coupling mechanism 816 configured to releasably couple to a drug holder 818 that contains the drug 802 therein. The drug holder 818 is a vial in this illustrated embodiment. FIG. 8 shows the drug holder 818 releasably coupled to the coupling mechanism 816.

The vial 818 includes a seal member 822 at a proximal end thereof. The seal member 822 is configured to provide a fluid tight seal such that the drug 802 is contained in the vial 818 until the seal provided by the seal member 822 is broken. The seal member 822 can have a variety of configurations, as will be appreciated by a person skilled in the art, such as by being a pierceable polymer septum or a foil layer. The seal member 110 can be protected from accidental puncturing or piercing before intended use with a removable protective member or stopper, such as a TE seal, etc.

The seal provided by the seal member 822 is configured to be broken by the coupling mechanism 816 of the drug delivery device 800 as a result of the vial 818 being releasably coupled thereto. The coupling mechanism 816 has a leading end 824 configured to puncture or pierce through the vial's seal member 822 in response to the vial 818 being loaded onto the drug delivery device 800. In other words, as the vial 818 and the drug delivery device 800 are coupled together, the coupling mechanism 816 punctures or pierces through the vial's seal member 822. The vial 818 can thus be fluid tight until the vial 818 is coupled to the drug delivery device 800, after which the drug 802 contained in the vial 818 can be delivered from the drug delivery device 800, as discussed further below. The leading end 824 of the coupling mechanism 816 can be configured to puncture or pierce the seal member 822 in any of a variety of ways. For example, the leading end 824 can be a pointed tip similar to a needle tip, as in this illustrated embodiment. For another example, the leading end 824 can include a sharp edge, e.g., around a perimeter of the coupling mechanism 816, to facilitate the puncturing or piercing of the vial's seal member 822 or can otherwise include a feature configured to facilitate the puncturing or piercing, such as by having one or more sharp extensions extending distally therefrom.

The drug holder 818 includes a handle 832 configured to facilitate manual handling of the drug holder 818 by a user. The handle 832 is at a distal end of the drug holder 818 opposite to the proximal end of the drug holder 818 at which the seal member 822 is located. The handle 814 may thus facilitate a user handling the drug holder 818 to couple the drug holder 818 to the coupling mechanism 816 of the drug delivery device 800. The handle 832 is a C-shaped member attached to the drug holder's distal end in this illustrated embodiment, but the handle 832 can have other configurations.

An exemplary drug holder can include a variety of features to facilitate sealing and storing a drug therein, as described herein and illustrated in the drawings. However, a person skilled in the art will appreciate that the drug holders can include only some of these features and/or can include a variety of other features known in the art. The vial 818 illustrated in FIG. 8 is merely intended to represent one exemplary embodiment.

The drug delivery device 800 does not need to be primed after the drug holder 818 has been releasably coupled thereto and prior to actuation of the drug delivery device 800 to cause drug delivery, which may ease use of the drug delivery device 800 by a user.

The body 804 of the drug delivery device 800 includes a propellant chamber 826, in which the propellant 820 is located. The propellant 820 is compressed air in this illustrated embodiment. The body 804 of the drug delivery device 800 also includes a pumping chamber 828, which is operatively coupled to the pumping mechanism 814. The coupling mechanism 816 is a tubular member with its inner lumen being in fluid communication with the pumping chamber 828. The dispensing head 806 is also in fluid communication with the pumping chamber 828. The drug 802 is configured to flow from the drug holder 818, through the coupling mechanism 816 into the pumping chamber 828, and then out the opening 810 formed in the dispensing head 806 in response to actuation of the actuator 812. The actuator 812 is configured to selectively prevent and allow fluid communication between the propellant chamber 826 and the pumping chamber 828. In an unactuated state, the actuator 812 prevents fluid communication between the propellant chamber 826 and the pumping chamber 828. In an actuated state, the actuator 812 allows fluid communication between the propellant chamber 826 and the pumping chamber 828. FIG. 8 shows the actuator 812 in the unactuated state. The actuator 812 can have a variety of configurations. For example, the actuator 812 can include a push-button valve configured to be selectively closed, corresponding to the unactuated state and the button not being pressed, and open, corresponding to the actuated state and the button being pressed.

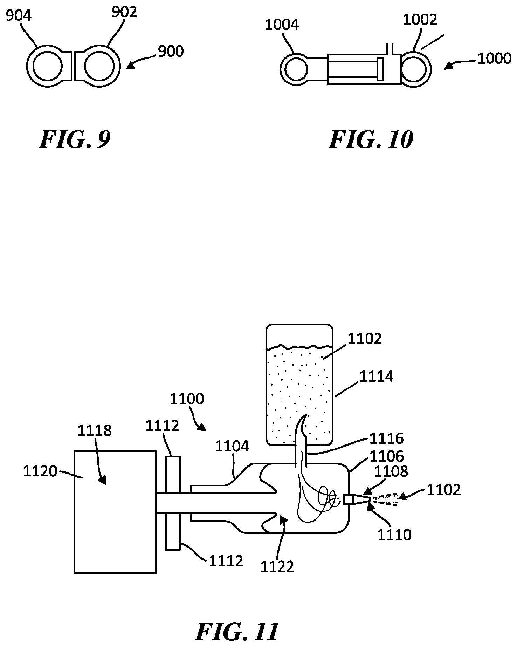

The pumping mechanism 814 is configured to be pumped by a user of the drug delivery device 800, e.g., moved back and forth (up and down in the view of FIG. 8) by hand. The pumping of the pumping mechanism 814 is configured to pressurize the pumping chamber 828. The pumping mechanism 814 includes a handle 830 configured to facilitate handling of the pumping mechanism 814 during pumping. The handle 830 in this illustrated embodiment includes a single finger ring, but the handle 830 can have other configurations. FIG. 9 illustrates another embodiment of a pumping mechanism handle 900 that includes a finger ring 902 and a thumb ring 904. FIG. 10 illustrates another embodiment of a pumping mechanism handle 1000 that includes a finger ring 1002 and a thumb ring 1004. The pumping mechanism handle rings in the embodiments of FIGS. 8 and 9 are stationary relative to a remainder of the pumping mechanism, whereas one ring 1004 of the pumping mechanism handle rings in the handle 1000 of FIG. 10 is slidably movable relative to the other ring 1002 of the pumping mechanism handle rings and a remainder of the pumping mechanism. The sliding movement of the pumping mechanism's ring 1004 creates pressurization in the pressure chamber similar to the sliding movement of the pumping mechanism 814 of FIG. 8, but the other, stationary ring 1002 may provide helpful leverage to the user during pumping.

Referring again to FIG. 8, in an exemplary method of using the drug delivery device 800, the drug holder 818 is coupled to the drug delivery device 800, e.g., to the coupling mechanism 816, after the pumping chamber 828 has been pressurized. The tip 808 is positioned in a nostril after the drug holder 818 has been coupled to the drug delivery device 800. The tip 808 can be positioned in the nostril before the drug holder 818 has been coupled to the drug delivery device 800, but the drug delivery device 800 may be more awkward to handle for most users and/or may result in patient discomfort if the drug holder 818 is coupled to the drug delivery device 800 after the tip 808 has been positioned in the nostril. After the pumping chamber 828 has been pressurized and the tip 808 has been positioned in a nostril of the patient, the actuator 812 is actuated. The actuation of the actuator 812 causes the propellant and pumping chambers 826, 828 to become in fluid communication with one another. The pressurized pumping chamber 818 will cause the propellant 820 in the propellant chamber 828 to rush into the pumping chamber 818 and thereby cause drug 802 that has entered the pumping chamber 828 to be propelled out of the opening 810 of the drug delivery device 800. The drug 802 exits through the opening 810 at a high enough velocity to be released as a spray due to the Venturi effect.

In the illustrated embodiment of FIG. 8, the drug delivery device 800 is configured to deliver the drug 802 into only one nostril of a patient, e.g., with the tip 808 positioned in the one nostril. In another embodiment, the drug delivery device 800 of FIG. 8 is configured to simultaneously deliver the drug 802 into both nostrils of a patient, similar to that discussed above regarding the drug delivery device 200 of FIG. 2.

FIG. 11 illustrates another exemplary embodiment of the drug delivery device 100 of FIG. 1. FIG. 11 illustrates a drug delivery device 1100 configured to expel a drug 1102 into a nose of a patient and to drive delivery of the drug 1102 using a propellant 1120. The drug delivery device 1100 of FIG. 11 is generally configured and used similar to the drug delivery device 800 of FIG. 8, e.g., includes a body 1104, a dispensing head 1106 including a tip 1108 with an opening 1110 therein, an actuator 1112, and a coupling mechanism 1116 configured to releasably couple to a drug holder 1114 that contains the drug 1102 therein and to puncture or pierce the drug holder 1114 in response to the drug holder 1114 being coupled thereto. However, unlike the drug delivery device 800 of FIG. 8, the drug delivery device 1100 of FIG. 11 does not include a pumping mechanism, and the drug holder 1114 is a blow-fill-seal (BFS) capsule.

The body 1104 includes a propellant chamber 1118 in which the propellant 1120 is located. The propellant 1120 is compressed air in this illustrated embodiment. The body 1104 of the drug delivery device 1100 also includes a swirl chamber 1122, which is defined by the dispensing head 1106. The coupling mechanism 1116 is a tubular member with its inner lumen being in fluid communication with the swirl chamber 1122. The drug 1102 is configured to flow from the drug holder 1114, through the coupling mechanism 1116 into the swirl chamber 1122, and then out the opening 1110 in response to actuation of the actuator 1112. The actuator 1112 is configured to selectively prevent and allow fluid communication between the propellant chamber 1118 and the swirl chamber 1122. In an unactuated state, the actuator 1112 prevents fluid communication between the propellant chamber 1118 and the swirl chamber 1122. In an actuated state, the actuator 1112 allows fluid communication between the propellant chamber 1118 and the swirl chamber 1122 such that the propellant 1120 and the drug 1102 swirl in the swirl chamber 1122 and thereby propel the drug 1102 through the opening 1110. The actuator 1112 can have a variety of configurations. For example, the actuator 1112 can include a spring valve configured to be selectively closed, corresponding to the unactuated state and the spring being in its biased position, and open, corresponding to the actuated state and the spring not being in its biased position. FIG. 11 shows the actuator 1112 in the actuated state with the propellant 1120 and the drug 1102 swirling in the swirl chamber 1122 and the drug 1102 spraying out the opening 1110. The drug 1102 exits through the opening 1110 at a high enough velocity to be released as a spray due to the Venturi effect.

In the illustrated embodiment of FIG. 11, the drug delivery device 1100 is configured to deliver the drug 1102 into only one nostril of a patient, e.g., with the tip 1108 positioned in the one nostril. In another embodiment, the drug delivery device 1100 of FIG. 11 is configured to simultaneously deliver the drug 1102 into both nostrils of a patient, similar to that discussed above regarding the drug delivery device 200 of FIG. 2.

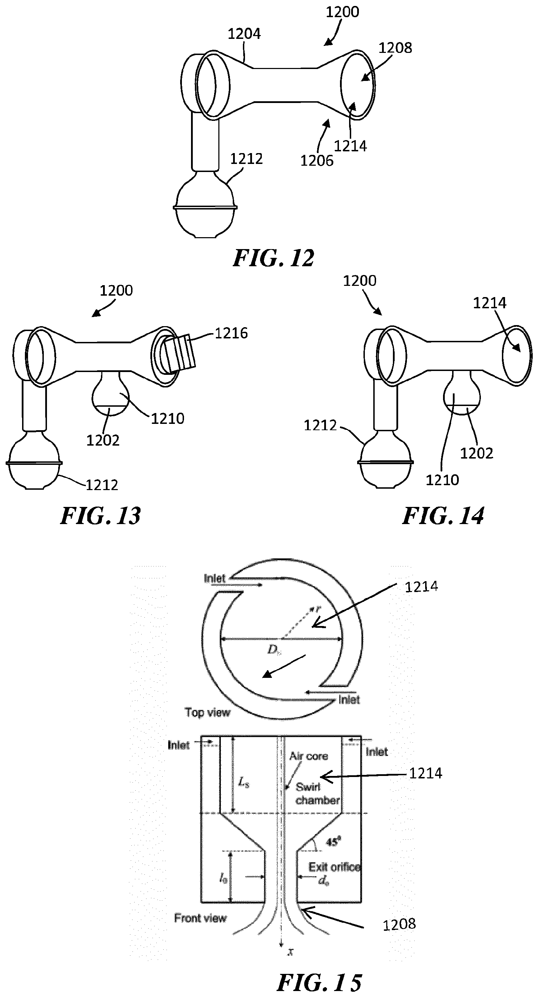

FIGS. 12-14 illustrate another exemplary embodiment of the drug delivery device 100 of FIG. 1. FIGS. 12-14 illustrate a drug delivery device 1200 configured to expel a drug 1202 into a nose of a patient and to drive delivery of the drug 1202 using a propellant. The drug delivery device 1200 of FIGS. 12-14 is generally configured and used similar to the drug delivery device 1100 of FIG. 11, e.g., includes a body 1204, a dispensing head 1206 with an opening 1208 therein, a coupling mechanism (obscured in FIGS. 12-14) configured to releasably couple to a drug holder 1210 that contains the drug 1202 therein and to puncture or pierce the drug holder 1210 in response to the drug holder 1210 being coupled thereto, a propellant chamber 1212, and a swirl chamber 1214 defined by the dispensing head 1206. However, unlike the drug delivery device 1100 of FIG. 11, the drug holder 1210 is a vial, and the opening 1208 is not in a tip of the dispensing head 1206 insertable into a nostril. Instead, the dispensing head 1206 is configured to abut the bottom of a patient's nose with the opening 1208 positioned under the patient's nostril. In other embodiments, the dispensing head 1206 can include a tip with the opening 1208 therein.

FIG. 12 illustrates the drug delivery device 1200 without the drug holder 1210 coupled thereto. FIGS. 13 and 14 illustrate the drug delivery device 1200 with the drug holder 1210 coupled thereto. Also, as shown in FIG. 13, the drug delivery device 1200 includes a removable protective member or cap 1216 that is configured to be removed just prior to use of the drug delivery device 1200. The protective member or cap 1216 can be replaced after use of the drug delivery device 1200. FIG. 13 shows that the drug holder 1210 has been coupled to the coupling mechanism prior to removal of the protective member or cap 1216. Removing the protective member or cap 1216 after the drug holder 1210 has been coupled to the coupling mechanism may help prevent premature drug delivery by keeping the opening 1208 covered until just before an intended time of drug delivery.

The propellant chamber 1212 in this illustrated embodiment defines the actuator configured to be actuated to begin drug delivery. The propellant 1120 is air in this illustrated embodiment. The propellant chamber 1212 includes a hollow squeezable body, which in this illustrated embodiment is a squeezable bulb. In an unactuated or unsqueezed state, there is not propellant or drug 1202 flow in the body 1204, including in the swirl chamber 1214. In an actuated or squeezed state, the propellant chamber 1212 causes propellant or drug 1202 flow in the body 1204 such that the propellant and the drug 1202 swirl in the swirl chamber 1214 and thereby propel the drug 1202 through the opening 1208. FIG. 15 illustrates flow in and out of the swirl chamber 1214. Flow in a swirl chamber is further discussed in Man Chiu Fung, "Experimental and numerical study of spray characteristics of nasal spray device," School of Aerospace, Mechanical and Manufacturing Engineering Science, Engineering and Technology Portfolio RMIT University, August 2013 (available at <https://researchbank.rmit.edu.au/eserv/rmit: 160720/Fung.pdf>), see, e.g., p. 9-11.

In the illustrated embodiment of FIGS. 12-14, the drug delivery device 1200 is configured to deliver the drug 1202 into only one nostril of a patient, e.g., with the tip 1208 positioned in the one nostril. In another embodiment, the drug delivery device 1200 of FIGS. 12-14 is configured to simultaneously deliver the drug 1202 into both nostrils of a patient, similar to that discussed above regarding the drug delivery device 200 of FIG. 2.

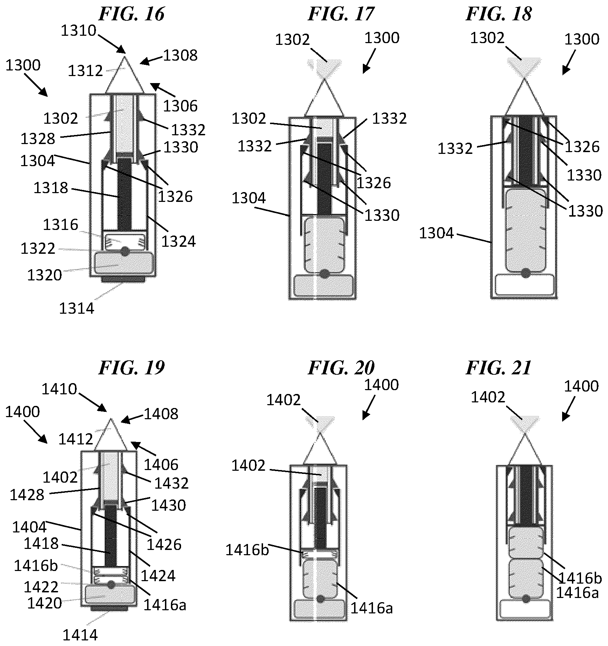

FIG. 16 illustrates another exemplary embodiment of the drug delivery device 100 of FIG. 1. FIG. 16 illustrates a drug delivery device 1300 configured to expel a drug 1302 into a nose of a patient and to drive delivery of the drug 1302 using a propellant. The drug delivery device 1300 of FIG. 16 is generally configured and used similar to the drug delivery device 200 of FIG. 2, e.g., includes a body 1304, a dispensing head 1306 including a tip 1308 that defines a spray chamber 1312 therein and that has an opening 1310 therein, and an actuator 1314. Additionally, similar to the embodiment of FIG, 7 that includes a bladder 716 containing a propellant therein, the drug delivery device 1300 of FIG. 16 includes a bladder 1316 that contains a propellant therein and that is a flexible member configured to expand, and the drug delivery device 1300 includes a plunger 1318 configured to push the drug 1302 proximally to be expelled out of the drug delivery device 1300 through the opening 1310. However, unlike the drug delivery device 700 of FIG. 7, the propellant in this illustrated embodiment includes a gas in a gas chamber 1320 of the drug delivery device 1300 that is configured to be released from the gas chamber 1320 to cause expansion of the bladder 1316. The expansion of the bladder 1316 is configured to press against the plunger 1318 in a proximal direction and thereby cause the drug 1302 to be delivered from the drug delivery device 1300.

The actuator 1314 of the drug delivery device 1300 includes a button that is configured to be actuated, e.g., pressed or otherwise moved. The actuation of the actuator 1314 is configured to cause a valve 1322 of the drug delivery device 1300 to move from a closed state to an open state. The valve 1322 is operatively coupled to the gas chamber 1320 and the bladder 1316. In the open state, the valve 1322 allows for fluid communication therethrough from the gas chamber 1320 to the bladder 1316 such that the gas in the gas chamber 1320 can enter into the bladder 1316 and cause expansion of the bladder 1316. In the closed state, the valve 1322 prevents fluid communication between the gas chamber 1320 and the bladder 1316.

In this illustrated embodiment, the actuator 1314 is configured to be actuated twice, with a first actuation of the actuator 1314 corresponding to a first stage of operation of the drug delivery device 1300 with drug delivery into one nostril of a patient and a second, subsequent actuation of the actuator 1314 corresponding to a second stage of operation of the drug delivery device 1300 with drug delivery into the other nostril of the patient. In such an embodiment, as shown in FIG. 17, the first actuation of the actuator 1314 is configured to release a first predetermined amount of the gas from the gas chamber 1314 into the bladder 1316 to cause a first expansion of the bladder 1316 and thus a first proximal movement of the plunger 1318 to push a first amount of the drug 1302 out of the drug delivery device 1300. The second actuation of the actuator 1314, as shown in FIG. 18, is configured to release a second predetermined amount of the gas from the gas chamber 1314 into the bladder 1316 to cause a second, further expansion of the bladder 1316 and thus a second proximal movement of the plunger 1318 to push a second amount of the drug 1302 out of the drug delivery device 1300. In an exemplary embodiment, the first and second predetermined amounts of gas are the same, and thus the first and second amounts of the drug 1302 are the same, such that a same sized dose of the drug 1302 is delivered out of the drug delivery device 1300 with each actuator 1314 actuation.

The drug delivery device 1300 includes engagable ramps configured to facilitate operation of the drug delivery device 1300 in stages. A distal member 1324 of the drug delivery device 1300 is movably disposed in the body 1304 and is operatively coupled to the bladder 1316. The distal member 1324 includes a proximal ramp 1326 thereon. The proximal ramp 1326 can include a plurality of protrusions positioned around a proximal perimeter of the distal member 1324, a single protrusion along a partial perimeter of the distal member 1324, or a single protrusion along a complete perimeter of the distal member 1324. A proximal member 1328 of the drug delivery device 1300 is disposed in the body 1304 at a fixed position relative thereto. The proximal member 1328 includes a first ramp 1330 thereon and a second ramp 1332 thereon that is located proximal to the first ramp 1330. The first and second ramps 1330, 1332 can have a variety of configurations, similar to the proximal ramp 1326. The positioning and number of the first and second ramps 1330, 1332 corresponds to the positioning and number of the proximal ramp 1326.

As shown in FIG. 17, when the drug delivery device 1300 is in an initial configuration before the first actuation of the actuator 1314, the proximal ramp 1326 is engaged with the first ramp 1330. As shown in FIG. 18, the first actuation of the actuator 1314 causes the proximal ramp 1326 to move proximally past the first ramp 1330 and engage the second ramp 1332, which stops the proximal movement of the bladder 1314 and the plunger 1318 in cooperation with the first predetermined amount of gas. As shown in FIG. 19, the second actuation of the actuator 1314 causes the proximal ramp 1326 to move proximally past the second ramp 1332 and engage a proximal interior surface of the body 1304, which stops the proximal movement of the bladder 1314 and the plunger 1318 in cooperation with the second predetermined amount of gas.

The bladder 1316 can be made from one or more materials configured to prevent the propellant from passing therethrough and that do not adversely affect the propellant (e.g., by causing the propellant to degrade during storage of the drug delivery device 1300). For example, the bladder 1316 can be made from one or more polymers, as will be appreciated by a person skilled in the art.

In the illustrated embodiment of FIG. 13, the drug delivery device 1300 is configured to deliver the drug 1302 into only one nostril of a patient, e.g., with the tip 1308 positioned in the one nostril. In another embodiment, the drug delivery device 1300 of FIG. 16 is configured to simultaneously deliver the drug 1302 into both nostrils of a patient, similar to that discussed above regarding the drug delivery device 200 of FIG. 2.

In the illustrated embodiment of FIG. 16, the drug delivery device 1300 is configured to deliver the drug 1302 into only one nostril of a patient, e.g., with the tip 1308 positioned in the one nostril. In another embodiment, the drug delivery device 1300 of FIG. 16 is configured to simultaneously deliver the drug 1302 into both nostrils of a patient, similar to that discussed above regarding the drug delivery device 200 of FIG. 2. In such embodiments, the ramps are eliminated such that one actuation of the actuator 1314 can simultaneously deliver the drug 1302 into two nostrils.

FIG. 19 illustrates another exemplary embodiment of the drug delivery device 100 of FIG. 1. FIG. 19 illustrates a drug delivery device 1400 configured to expel a drug 1402 into a nose of a patient and to drive delivery of the drug 1402 using a propellant. The drug delivery device 1400 of FIG. 19 is generally configured and used similar to the drug delivery device 1300 of FIG. 16, e.g., includes a body 1404, a dispensing head 1406 including a tip 1408 that defines a spray chamber 1412 therein and that has an opening 1410 therein, an actuator 1414, a plunger 1418, a gas chamber 1420, a valve 1422, a distal member 1424 including a proximal ramp 1426, and a proximal member 1428 including a first ramp 1430 and a second ramp 1432. However, unlike the embodiment of FIG. 16 that includes a single bladder 1316, the embodiment of FIG. 19 includes distal and proximal bladders 1416a, 1416b . In a first stage of operation of the drug delivery device 1400, as shown in FIG. 20, the distal bladder 1416a expands to move the plunger 1418. In a second stage of operation of the drug delivery device 1400, as shown in FIG. 21, the proximal bladder 1416b expands to move the plunger 1418.

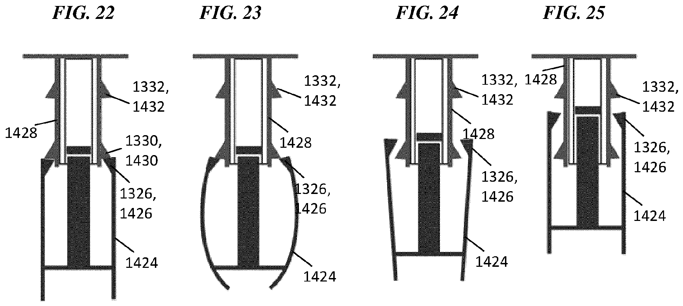

FIGS. 22-25 further illustrate interaction of the proximal ramp 1326, 1426 with the first ramp 1330, 1430 of the embodiments of FIGS. 16-21. FIG. 22 illustrates the initial configuration of FIGS. 16 and 19. FIG. 23-25 occur sequentially during the first stage of operation as the drug delivery device 1300, 1400 moves toward the end of the first stage of operation shown in FIGS. 17 and 20. The proximal ramp 1326, 1426 similarly interacts with the second ramp 1332, 1432.

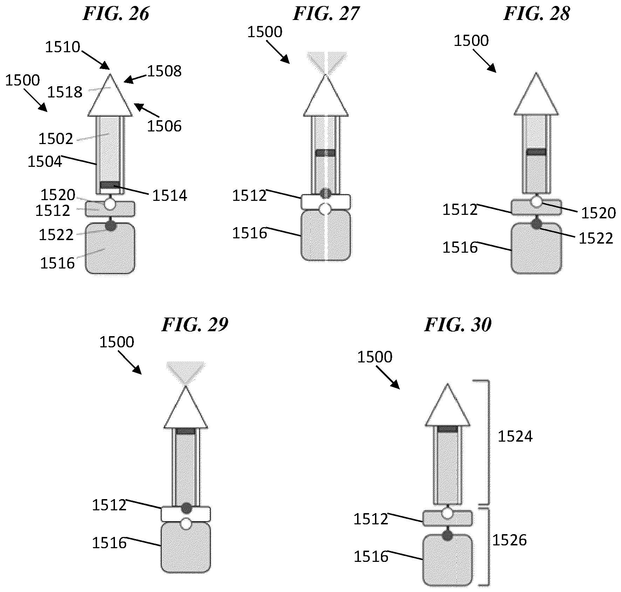

FIG. 26 illustrates another exemplary embodiment of the drug delivery device 100 of FIG. 1. FIG. 26 illustrates a drug delivery device 1500 configured to expel a drug 1502 into a nose of a patient and to drive delivery of the drug 1502 using a propellant. The drug delivery device 1500 of FIG. 26 is generally configured and used similar to the drug delivery device 500 of FIG. 5, e.g., includes a body 1504, a dispensing head 1506 including a tip 1508 defining a spray chamber 1518 and having an opening 1510 therein, and a stopper 1514. However, unlike the embodiment of FIG. 5, the drug delivery device 1500 of FIG. 26 is configured to be actuated twice to deliver the drug 1502 in two stages of operation.

To facilitate the two stages of operation, the drug delivery device 1500 includes a first container 1512 located outside of the body 1504 and containing a first propellant therein, a second container 1516 located outside of the body 1504 and containing a second propellant therein that is at high pressure, a first valve 1520 located between the first and second containers 1512, 1516, and a second valve 1522 located between the first container 1512 and the body 1504. In an initial configuration of the drug delivery device 1500, shown in FIG. 26, the first valve 1520 is closed, and the second valve 1522 is open. In a first stage of operation of the drug delivery device 1500, as shown in FIG. 27, the drug delivery device 1500 is actuated by squeezing the first container 1512. In response, the gas in the first container 1512 exits the first container 1512, passes through the first valve 1520 (which has moved to an open state due to the squeezing of the first container 1512) and a tubular conduit in fluid communication with the first container 1512 and a distal portion of an internal cavity of the body 1504 that is distal to the stopper 1514, and into the distal portion of the body's internal cavity to drive the stopper proximally 1514 and cause the drug 1502 to exit through the opening 1510. The squeezing stops, which causes the first valve 1520 to move from the open state to a closed state and the second valve 1522 to move from a closed state to an open state, as shown in FIG. 28, such that high pressure gas from the second container 1516 enters the first container 1512. In a second stage of operation of the drug delivery device 1500, as shown in FIG. 29, the drug delivery device 1500 is actuated by squeezing the first container 1512, which causes drug delivery similar to that discussed above regarding the first stage of operation. The squeezing stops, which causes the first valve 1520 to move from the open state to a closed state and the second valve 1522 to move from a closed state to an open state, as shown in FIG. 30, such that high pressure gas from the second container 1516 enters the first container 1512. A proximal, disposable portion 1524 of the drug delivery device 1500 depleted of the drug 1502 can then be disposed of for recycling or as medical waste, and a distal, reusable portion 1526 of the drug delivery device 1500 can be coupled to another proximal portion including another drug supply. In other embodiments, the entire drug delivery device 1500 can be disposed.

Embodiments of nasal drug delivery devices disclosed herein can be designed to be disposed of after a single use, or they can be designed to be used multiple times. In either case, in at least some embodiments, the drug delivery device can be reconditioned for reuse after at least one use. Reconditioning can include any combination of the steps of disassembly of the drug delivery device, followed by cleaning or replacement of particular pieces and subsequent reassembly. In particular, the drug delivery device can be disassembled, and any number of the particular pieces or parts of the drug delivery device can be selectively replaced or removed in any combination. Upon cleaning and/or replacement of particular parts, the drug delivery device can be reassembled for subsequent use either at a reconditioning facility, or by a health care provider immediately prior to use. A person skilled in the art will appreciate that reconditioning of a drug delivery device can utilize a variety of techniques for disassembly, cleaning/replacement, and reassembly. Use of such techniques, and the resulting reconditioned drug delivery device, are all within the scope of the present application.

The present disclosure has been described above by way of example only within the context of the overall disclosure provided herein. It will be appreciated that modifications within the scope of the claims may be made without departing from the overall scope of the present disclosure.

## Claims

1. A drug delivery device (200, 500), comprising:
a tip configured to be positioned at a nose of a patient, the tip having an opening therein;
a propellant chamber containing a propellant therein;
a drug chamber containing a drug (202, 502) therein;
a stopper (208, 516) positioned between the propellant chamber and the drug chamber; and
an actuator (212) configured to be actuated to cause the propellant to propel movement of the stopper in a direction toward the drug chamber and thereby propel the drug (202, 502) out of the opening.

2. The device of claim 1, further comprising a body that defines an internal cavity, the internal cavity defining the propellant chamber and the drug chamber.

3. The device of claim 1, further comprising a body that defines an internal cavity, the internal cavity defining the drug chamber and having the stopper (508) disposed therein;
a container (514) defining the propellant chamber; and
a tubular conduit (520) extending between the container and the body, the conduit providing fluid communication between the internal cavity and the propellant chamber.

4. The device of any preceding claim, further comprising a first seal member (216, 512) that separates the tip from the drug chamber prior to the actuation of the actuator;
wherein the propelling of the drug is configured to break the first seal member (216, 512).

5. The device of any preceding claim, wherein the tip, the propellant chamber, the drug chamber, and the stopper are longitudinally aligned with one another.

6. The device of any of claims 1-4, wherein the propellant chamber and the drug chamber are not longitudinally aligned with one another.

7. The device of any preceding claim, wherein the propellant includes at least one of a gas and a material substance configured to produce energy that causes movement of the drug out of the opening.

8. The device of any preceding claim, further comprising a second seal member (222) that separates the propellant chamber into a first area, containing a first component of the propellant, and a second area, containing a second component of the propellant, prior to the actuation of the actuator such that the first and second components of the propellant are separated from one another prior to the actuation of the actuator.

9. The device of claim 8, wherein the actuation of the actuator (212) is configured to automatically break the second seal member.

10. The device of claim 8, wherein the first component includes vinegar, and the second component includes sodium bicarbonate.

11. The device of claim 1, wherein the drug is one of ketamine, esketamine, naloxone, and sumatriptan.

## Patentansprüche

1. Arzneimittelabgabevorrichtung (200, 500), umfassend:
eine Spitze, die konfiguriert ist, um an einer Nase eines Patienten positioniert zu werden, wobei die Spitze eine Öffnung darin aufweist;
eine Treibmittelkammer, die ein Treibmittel darin enthält;
eine Arzneimittelkammer, die ein Arzneimittel (202, 502) darin enthält;
einen Stopfen (208, 516), der zwischen der Treibmittelkammer und der Arzneimittelkammer angeordnet ist; und
einen Aktuator (212), der konfiguriert ist, um betätigt zu werden, um zu bewirken, dass das Treibmittel eine Bewegung des Stopfens in eine Richtung zu der Arzneimittelkammer hin vorantreibt und dadurch das Arzneimittel (202, 502) aus der Öffnung vorantreibt.

2. Vorrichtung nach Anspruch 1, ferner umfassend einen Körper, der einen Innenhohlraum definiert, wobei der Innenhohlraum die Treibmittelkammer und die Arzneimittelkammer definiert.

3. Vorrichtung nach Anspruch 1, ferner umfassend einen Körper, der einen Innenhohlraum definiert, wobei der Innenhohlraum die Arzneimittelkammer definiert und den darin angeordneten Stopfen (508) aufweist;
einen Behälter (514), der die Treibmittelkammer definiert; und
eine röhrenförmige Leitung (520), die sich zwischen dem Behälter und dem Körper erstreckt, wobei die Leitung eine Fluidverbindung zwischen dem Innenhohlraum und der Treibmittelkammer bereitstellt.

4. Vorrichtung nach einem der vorstehenden Ansprüche, ferner umfassend ein erstes Dichtungselement (216, 512), das die Spitze vor der Betätigung des Aktuators von der Arzneimittelkammer trennt;
wobei das Vorantreiben des Arzneimittels konfiguriert ist, um das erste Dichtungselement (216, 512) zu durchbrechen.

5. Vorrichtung nach einem der vorstehenden Ansprüche, wobei die Spitze, die Treibmittelkammer, die Arzneimittelkammer und der Stopfen in Längsrichtung zueinander ausgerichtet sind.

6. Vorrichtung nach einem der Ansprüche 1 bis 4, wobei die Treibmittelkammer und die Arzneimittelkammer nicht in Längsrichtung zueinander ausgerichtet sind.

7. Vorrichtung nach einem der vorstehenden Ansprüche, wobei das Treibmittel mindestens eines von einem Gas und einer materiellen Substanz einschließt, die konfiguriert ist, um Energie zu erzeugen, die eine Bewegung des Arzneimittels aus der Öffnung heraus bewirkt.

8. Vorrichtung nach einem der vorstehenden Ansprüche, ferner umfassend ein zweites Dichtungselement (222), das die Treibmittelkammer vor der Betätigung des Aktuators in einen ersten Bereich, der eine erste Komponente des Treibmittels enthält, und einen zweiten Bereich, der eine zweite Komponente des Treibmittels enthält, unterteilt, so dass die erste und zweite Komponente des Treibmittels vor der Betätigung des Aktuators voneinander getrennt sind.

9. Vorrichtung nach Anspruch 8, wobei die Betätigung des Aktuators (212) konfiguriert ist, um das zweite Dichtungselement automatisch zu durchbrechen.

10. Vorrichtung nach Anspruch 8, wobei die erste Komponente Essig einschließt und die zweite Komponente Natriumbicarbonat einschließt.

11. Vorrichtung nach Anspruch 1, wobei das Arzneimittel eines von Ketamin, Esketamin, Naloxon oder Sumatriptan ist.

## Revendications

1. Dispositif d'administration de médicaments (200, 500), comprenant :
un embout conçu pour être positionné au niveau d'un nez d'un patient, l'embout ayant une ouverture en son sein ;
une chambre d'agent de propulsion contenant un agent de propulsion en son sein ;
une chambre à médicaments contenant un médicament (202, 502) en son sein ;
un bouchon (208, 516) placé entre la chambre d'agent de propulsion et la chambre à médicaments ; et
un actionneur (212) conçu pour être actionné pour amener l'agent de propulsion à provoquer un mouvement du bouchon dans une direction vers la chambre à médicaments et ainsi propulser le médicament (202, 502) hors de l'ouverture.

2. Dispositif selon la revendication 1, comprenant en outre un corps qui définit une cavité interne, la cavité interne définissant la chambre d'agent de propulsion et la chambre à médicaments.

3. Dispositif selon la revendication 1, comprenant en outre un corps qui définit une cavité interne, la cavité interne définissant la chambre à médicaments et ayant le bouchon (508) disposé en son sein ;
un récipient (514) définissant la chambre d'agent de propulsion ; et
un conduit tubulaire (520) s'étendant entre le récipient et le corps, le conduit fournissant une communication fluidique entre la cavité interne et la chambre d'agent de propulsion.

4. Dispositif selon l'une quelconque revendication précédente, comprenant en outre un premier élément d'étanchéité (216, 512) qui sépare l'embout de la chambre à médicaments avant l'actionnement de l'actionneur ;
dans lequel l'agent de propulsion du médicament est conçu pour briser le premier élément d'étanchéité (216, 512).

5. Dispositif selon l'une quelconque revendication précédente, dans lequel l'embout, la chambre d'agent de propulsion, la chambre à médicaments et le bouchon sont alignés longitudinalement les uns par rapport aux autres.

6. Dispositif selon l'une quelconque des revendications 1 à 4, dans lequel la chambre d'agent de propulsion et la chambre à médicaments ne sont pas alignées longitudinalement l'une par rapport à l'autre.

7. Dispositif selon l'une quelconque revendication précédente, dans lequel l'agent de propulsion comporte au moins l'un parmi un gaz et une substance matérielle conçue pour produire de l'énergie qui provoque un mouvement du médicament hors de l'ouverture.

8. Dispositif selon l'une quelconque revendication précédente, comprenant en outre un second élément d'étanchéité (222) qui sépare la chambre d'agent de propulsion en une première zone, contenant un premier composant de l'agent de propulsion, et une seconde zone, contenant un second composant de l'agent de propulsion, avant l'actionnement de l'actionneur, de telle sorte que les premier et second composants de l'agent de propulsion sont séparés l'un de l'autre avant l'actionnement de l'actionneur.

9. Dispositif selon la revendication 8, dans lequel l'actionnement de l'actionneur (212) est conçu pour briser automatiquement le second élément d'étanchéité.

10. Dispositif selon la revendication 8, dans lequel le premier composant comporte du vinaigre, et le second composant comporte du bicarbonate de sodium.

11. Dispositif selon la revendication 1, dans lequel le médicament est l'un parmi la kétamine, l'eskétamine, le naloxone et le sumatriptan.
